Europäisches Patentamt

(19) European Patent Office

Office européen des brevets

(11) Numéro de publication : **0 304 436 B1**

## (12) FASCICULE DE BREVET EUROPEEN

(45) Date de publication du fascicule du brevet :
**29.05.91 Bulletin 91/22**

(51) Int. Cl.⁵ : **A61M 1/30**

(21) Numéro de dépôt : **87905936.8**

(22) Date de dépôt : **10.09.87**

(86) Numéro de dépôt international :
**PCT/BE87/00013**

(87) Numéro de publication internationale :
**WO 88/01880 24.03.88 Gazette 88/07**

(54) APPAREILLAGE POUR L'HEMODIALYSE A UNE SEULE AIGUILLE.

(30) Priorité : **10.09.86 BE 217142**
**17.10.86 BE 217301**

(43) Date de publication de la demande :
**01.03.89 Bulletin 89/09**

(45) Mention de la délivrance du brevet :
**29.05.91 Bulletin 91/22**

(84) Etats contractants désignés :
**AT BE CH DE FR GB IT LI LU NL SE**

(56) Documents cités :
**EP-A-00 132 210**
**EP-A-01 048 95**

(56) Documents cités :
**EP-A-01 058 45**
**DE-A- 2 236 433**
**FR-A-22 421 12**
**FR-A-22 581 92**

(73) Titulaire : **HOMBROUCKX, Remi O.J.**
**Hogerluchtstraat, 6**
**B-9600 Ronse (BE)**

(72) Inventeur : **HOMBROUCKX, Remi O.J.**
**Hogerluchtstraat, 6**
**B-9600 Ronse (BE)**

(74) Mandataire : **Fobe, Edouard et al**
**Bureau VANDER HAEGHEN 63, Avenue de la Toison d'Or**
**B-1060 Bruxelles (BE)**

## Description

La présente invention est relative à un appareillage pour l'hémodialyse à une seule aiguille, dans lequel un volume prédéterminé de sang provenant d'une fistule, d'une veine ou d'une artère est alternativement retiré du corps du patient au moyen d'une pompe, pour être épuré dans un rein artificiel et ensuite retourné au patient au même endroit de ponction par une seule et même aiguille.

L'appareillage de dialyse comprend :
– une aiguille ou un cathéter,
– un tronçon de tuyau entre l'aiguille et la pompe,
– le rein artificiel,
– une chambre d'expansion constituée d'un récipient fermé pourvu d'une conduite d'amenée et d'évacuation du sang et muni à la partie supérieure d'une conduite apicale d'air et munie quelques millimètres plus bas d'une ligne d'infusion permettant de rincer le rein, d'administrer un médicament et d'adapter la hauteur du niveau de sang à l'aide d'air sous pression, et
– un dispositif de mesure et de contrôle de la pompe.

L'hémodialyse trouve sa principale application en néphrologie pour soigner des insuffisances rénales aiguës ainsi que chroniques. Ce traitement consiste à éliminer du sang des substances toxiques dans un circuit externe à l'aide d'un rein artificiel basé sur le principe de la diffusion des substances toxiques à travers une membrane semi-perméable.

Les patients doivent se raccorder 2 à 3 fois par semaine à un rein artificiel semi-automatique, à des intervalles compris généralement entre deux à trois jours en moyenne. Ils restent reliés à un rein artificiel pendant plusieurs heures à l'aide d'une aiguille-shunt ou cathéter et des conduites en matière synthétique.

Dans la dialyse classique le sang s'écoule dans une direction déterminée de part en part d'un rein artificiel, à savoir d'un côté artériel vers un côté veineux. Le sang est retiré du système circulatoire du patient du côté artériel, par le cathéter ou par l'aiguille de dialyse, refoulé ensuite à l'aide d'une pompe dans un sens déterminé dans le rein artificiel, et aboutit dans un vase d'expansion dans lequel la pression est enregistrée et les bulles d'air rassemblées pour retourner ensuite dans le système circulatoire du patient, selon les modes suivants : s'il s'agit d'un système à deux aiguilles, le sang purifié est refoulé d'emblée par un second cathéter ou aiguille reliée au côté veineux ou s'il s'agit d'un système uniponctural, le sang purifié est retourné par une seconde pompe ou par un système à clapets, vers la première aiguille pourvue d'un embranchement en Y.

Le premier système à une aiguille est décrit par Kopp et al dans un article intitulé "Single needle dialysis" et publié dans "Trans. Am. Soc. Art. Intern. Organs n° 18, pages 75-80, 1972

Ce système fait l'objet du Reissue américain n° 29.346 et du brevet américain n° 3.830.234.

Le problème posé consiste à aspirer et à retourner le sang au même endroit. Cela implique des difficultés techniques quant à la recirculation du sang filtré mais procure l'avantage incontestable qu'il suffit de n'introduire qu'une seule aiguille.

Ce système comporte un seul segment de pompe et deux vannes de solénoïde sur la ligne artérielle et veineuse de sang.

Les vannes sont articulées tour à tour.

On pompe le sang du système circulatoire du patient, en particulier à partir d'une artère jusqu'à ce qu'une pression prédéterminée soit atteinte dans la chambre veineuse. Une fois que cette pression est atteinte, on obture la ligne artérielle et on ouvre la ligne veineuse.

La pompe tourne de manière continue dans la même direction. De cette manière on refoule le sang par la même aiguille au même endroit dans le système circulatoire du patient.

En raison du fait que le cycle est régulé par le fait d'atteindre une certaine pression et ensuite par l'écoulement d'un certain temps, on désigne cette régulation généralement par le système pression-temps à une seule aiguille. En 1979, Ahmad propose une modification, en faisant ouvrir et obturer les vannes à des intervalles de temps déterminé. Il jugea utile d'inclure trois coussinets compressibles de 10 ml chacun dans le système circulatoire artériel. Un débit sanguin de 200 à 300 ml/min et une période d'obturation de 5 secondes étaient optimal. Outre l'avantage d'une ponction unique, ce système maintient la pression veineuse naturelle lors de la restitution du sang.

Puisque la pompe fonctionne sans interruption, on obtient, si la ligne artérielle est obturée, une dépression dans la ligne artérielle comprise entre la pompe et le clapet. Il se forme ainsi une recirculation accrue ainsi qu'un danger d'entraîner de l'air dans les conduites. Cela entraîne la formation de mousse dans la chambre veineuse, ce qui provoque la coagulation du sang dans cette chambre.

On note comme inconvénient une ultrafiltration excessive lorsqu'on souhaite maintenir le débit à un niveau suffisamment important.

Ce système de pression-temps ne peut être utilisé que rarement chez un patient pourvu d'une fistule à l'aine et souffrant d'hypertension, en raison de la pression naturelle trop élevée dans la fistule.

Une méthode de dialyse très commode est l'hémodialyse à une seule aiguille pression-pression. Elle a été décrite dès 1973 par le Professeur Dr. S. RINGOIR et ses collaborateurs dans une publication intitulée "New pumpsystem for one needle hemodialysis" publiée par EDTA Abstracts, Vienne 200, 1973.

Il s'agit également d'un système à une seule aiguille dans lequel l'amenée du sang dans le circuit extracorporel s'effectue à l'aide d'une pompe à double tête (BELLCO 760 B). La conduite des pompes, qui sont enclenchées alternativement repose sur une commande à pression-pression.

Pendant la phase artérielle, du sang veineux est retiré du corps du patient, au moyen d'une pompe artérielle et conduit vers la chambre d'expansion veineuse, qui est destinée à réaliser une accumulation déterminée de sang. La phase artérielle est poursuivie, jusqu'à ce qu'une pression de consigne soit atteinte dans la chambre veineuse. La pompe artérielle est arrêtée et bloque ainsi la ligne artérielle. La pompe veineuse entre immédiatement en fonctionnement et refoule le sang dans le système circulatoire du patient par la même aiguille, et cela jusqu'à ce qu'une pression minimale de consigne soit atteinte. Ce réglage des pressions dépend entre autres du degré d'ultrafiltration que l'on souhaite obtenir. Une variante à ce sujet consiste à comparer les pressions de consigne avec les pressions dans la chambre artérielle, comme décrit par exemple dans le document US-A-4,643,714.

Ce système a été amélioré par le Dr. R. HOMBROUCKX et al par l'insertion d'une chambre d'expansion artérielle entre la pompe artérielle et le dialyseur.

On connaît aussi, comme décrit dans le document FR-A-2258 192, un récipient d'accumulation de sang destiné à être branché dans un circuit sanguin extracorporel d'un appareil de dialyse à aiguille unique, en amont d'une pompe à sang.

Le récipient possède branché sur une paroi de fond, une entrée et une sortie de sang. Un volume de gaz est délimité dans le récipient au-dessus d'un volume variable de sang et est relié à un réservoir de gaz par une conduite apicale. Le volume de gaz permet d'assumer en amont de la pompe, une pression suffisante pour empêcher un affaissement d'un vaisseau sanguin du patient.

Depuis l'utilisation de reins artificiels à fibres creuses, qui possèdent une compliance faible, c'est-à-dire qui contiennent un volume de sang prédéterminé invariable quelque soit la pression la chambre d'expansion artérielle et le récipient d'accumulation de sang ont procuré les avantages importants suivants :

1. accouplement aisé du patient à l'appareillage de dialyse puisqu'une seule ponction est nécessaire ;

2. flux continu avec pour conséquence une meilleure dialyse ;

3. prévention de formation de mousse, par le fait que pendant la phase veineuse, le sang n'est pas aspiré au travers du rein mais plutôt refoulé au travers du rein, grâce à l'intervention de la chambre d'expansion artérielle sous pression ;

4. élimination de mousse par le fait que la conduite d'évacuation du sang est prévue en dessous ;

5. réduction de la recirculation ; bien que la perte par recirculation comporte encore 10% ;

6. réglage du débit par la réduction du nombre d'enclenchements et arrêts des pompes, et donc réduction des "temps morts" entre l'arrêt d'une des pompes et l'enclenchement de l'autre ;

7. ultrafiltration parfaitement réglable, tant minimale que maximale ;

8. possibilité d'utilisation avec toutes les fistules, également celles présentant une pression naturelle élevée, telles que les fistules de l'aine et la veine "Safena", à hauteur de l'avant bras.

La chambre d'expansion est par exemple, un flacon pour circulation du sang, tel que décrit dans le document FR-A-2258192. Ce flacon être branché dans le circuit d'un appareil de dialyse à aiguille unique, en vue d'éviter la sous-alimentation de la pompe.

Le flacon possède une entrée et une sortie de sang ménagées dans le fond du flacon pour la liaison à un circuit formé d'une boucle fermée. Un coussin d'air délimité dans le flacon au-dessus du volume de sang est relié par une tube de transmission de pression à un compteur de pression commandant la pompe.

L'inconvénient le plus important de l'hémodialyse à une seule aiguille par pression-pression réside dans la complexité de l'appareillage, de sorte que la dialyse à domicile reste laborieuse.

Ces deux pompes exigent un système de commande complexe et sensible sur base de la création d'une pression déterminée. La variabilité et la précision sévère du programme de conduite de consigne rend la mise en oeuvre de la dialyse par des proches parents du patient difficile à domicile. La manipulation des appareils de dialyse connus exige un entraînement de plusieurs mois.

Le but de la présente invention est donc la réalisation d'un appareillage de dialyse de conduite plus simple.

On connaît également par le document EP-A-0132210 un procédé de plasmaphérèse au cours duquel du sang d'un donneur est prélevé grâce à une aiguille classique de prise de sang et est traité par filtration dans un appareil de plasmaphérèse comportant une membrane semi-perméable. Une pompe assure d'un côté de la membrane une pression suffisante pour séparer par filtration du plasma au travers de la membrane.

Le sang traité par plasmaphérèse est accumulé dans un récipient souple muni d'une poche gonflable. Le sang est réinjecté au donneur, au cours d'une phase dite de retour, en repassant, en sens inverse, par l'appareil à membrane, la pression d'entrée du sang étant assurée par une poche gonflable agissant sur le récipient souple.

On décrit dans ce document, également un appareillage utilisable pour la plasmaphérèse de don.

D'autres applications sont envisagées, par exemple l'hémofiltration moyennant la réinjonction d'un liquide de substitution asservie à la quantité de liquide filtré éliminée au travers de la membrane semi-perméable et recueillie dans un compartiment oral relié à l'extérieur par une seule conduite de soutirage. L'échange de matière liquide/liquide et en particulier la dialyse, n'est pas suggéré dans ce document.

Comme caractérisé dans les revendications ci-jointes, le retrait d'une quantité prédéterminée de sang du corps du patient et le déplacement au travers du rein artificiel dans la chambre d'expansion d'une part, et le retour du sang épuré dans le corps du patient d'autre part, par déplacement du sang en sens opposé le long d'une et même ligne sanguine, sont réalisés à l'aide d'une pompe réversible, dont le sens de rotation est commandé automatiquement d'une manière programmée à l'aide d'un dispositif de régulation et de mesure.

Ce dispositif de régulation et de mesure peut être un dispositif quelconque, par exemple un dispositif de mesure de pression, poids, volume, débit ou autre.

Ce rein artificiel est relié d'un côté à l'aide d'une pompe et une seule aiguille ou cathéter au système circulatoire du patient et de l'autre côté relié à une chambre d'expansion comprenant un récipient muni d'une conduite apicale d'air destinée à jouer le rôle d'une ligne de pression et quelques millimètres plus bas, d'une ligne d'infusion pour le rinçage du rein, l'administration de médicaments et le réglage du niveau du sang à l'aide de la quantité d'air injectée.

Les fluctuations de pression dans la chambre d'expansion et la chambre de compliance sont détectées par un dispositif de mesure de pression.

Par la mise en place d'une seule pompe, par la suppression des vannes occlusives et de l'appareillage superflu de mesure et de régulation, on a réduit sensiblement le coût de l'appareillage ainsi que le nombre de pièces d'accouplement dans la ligne sanguine de l'appareillage de dialyse.

Le montage, le démontage, le nettoyage, l'entretien et le contrôle de l'appareillage de dialyse est ainsi sensiblement facilité. La simplicité du fonctionnement alternatif rend l'utilisation de l'appareillage facile.

Puisque le contenu du volume interne de la ligne sanguine, du segment de pompe, du rein artificiel et de la chambre d'expansion est très réduit, la disposition de montage de l'appareillage de dialyse selon l'invention peut être considérée comme très sûre.

En cas de dérangement très grave, la pompe est arrêtée, ce qui stoppe immédiatement l'amenée de sang.

Par ce fait même, le contenu du rein artificiel et le contenu instantané de la chambre d'expansion peut être perdu dans le pire des cas. Même alors le système est considéré comme sûr, parce que ce contenu comporte tout au plus 200 ml de sang.

Par le fait que le sang traverse deux fois d'affilée le rein artificiel, on observe un net accroissement de l'efficacité de la dialyse.

En s'éloignant du point de ponction, de la pompe et du rein artificiel, la ligne sanguine se dirige vers la chambre d'expansion, qui est reliée par une conduite de pression à une chambre de compliance, afin d'atténuer les fluctuations de pression provoquées par le contenu variable de sang dans la chambre d'expansion. Ces fluctuations sont mesurées par un dispositif de mesure de la pression avec valeurs de consigne réglables, et servant à modifier tour à tour le sens de rotation de la pompe et donc le sens de circulation du sang dans la ligne de sang.

Selon une variante l'amenée d'une quantité prédéterminée de sang issu du système circulatoire du patient ainsi que le déplacement au travers du rein artificiel vers une chambre d'expansion s'effectue avec une première tête de pompe, tandis que le retour du sang épuré dans le système circulatoire s'effectue après une seconde épuration au travers du rein artificiel, dans le sens opposé à l'aide d'une seconde pompe, qui est enclenchée alternativement, la régulation de la pompe double en fonctionnement alternatif reposant, comme connu, sur une régulation pression-pression.

Cette variante offre l'avantage qu'elle empêche toute recirculation du volume de sang emprisonné dans le segment de pompe et dans les lignes sanguines entre l'aiguille et la pompe d'une part, et la pompe et le rein artificiel d'autre part, de sorte qu'aucun volume de sang non traité n'est plus retourné dans le système circulatoire du patient, et ce quelle que soit la longueur et la section des lignes sanguines et du segment de pompe.

Ce sang prélevé est de préférence refoulé à l'aide de la première pompe vers le rein artificiel et de là vers la chambre d'expansion, qui est destinée à réaliser une accumulation déterminée de sang sous pression. Cette première phase est poursuivie jusqu'à ce qu'une pression préalablement choisie est atteinte dans la chambre d'expansion.

Dans une seconde phase, une seconde pompe pompe le sang depuis la chambre d'expansion vers le rein artificiel et par la même aiguille, vers le système circulatoire du patient.

Ces particularités et détails de l'invention, ainsi que d'autres apparaîtront au cours de la description détaillée suivante de trois formes de réalisation différentes de l'invention, en faisant référence aux dessins suivants qui les illustrent schématiquement.

Dans ces dessins :
– la figure 1 est une illustration schématique d'une disposition de la première forme de réalisation de l'appareil de dialyse selon l'invention ; les flèches indiquent le sens du sang artériel pendant la phase artérielle ;

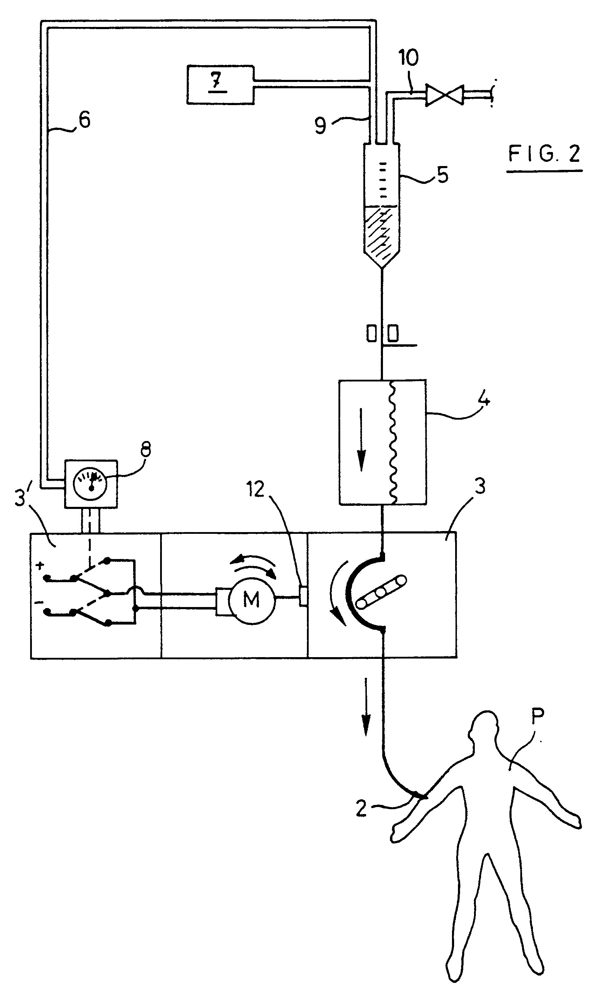

– la figure 2 illustre le fonctionnement de l'appareil illustré à la figure 1, pendant la phase veineuse ;

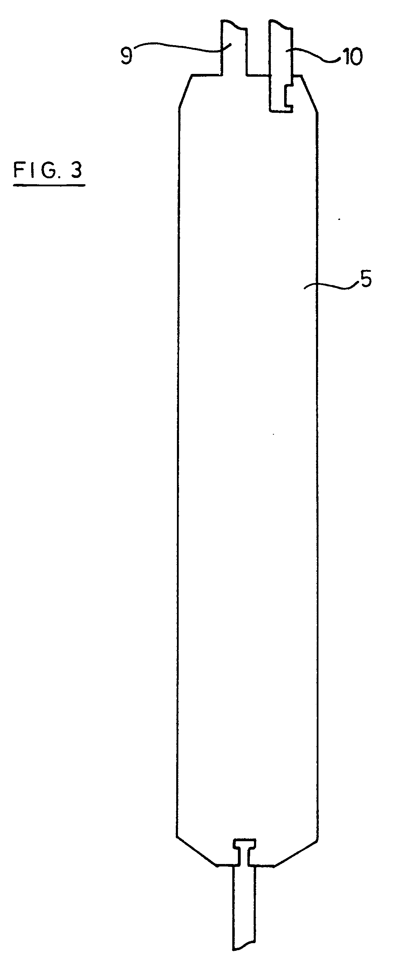

– la figure 3 est une coupe verticale d'une chambre d'expansion avec fond d'évacuation comprise ; et

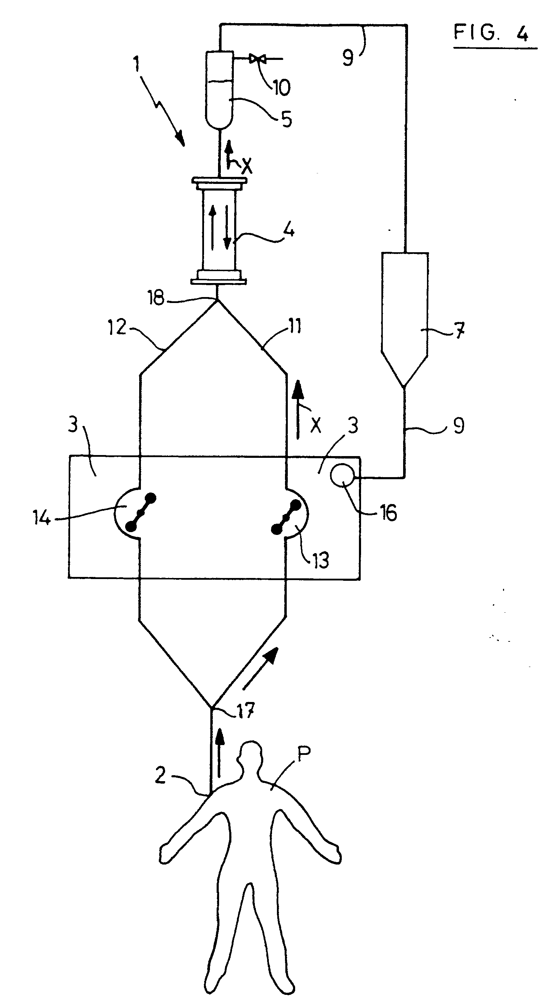

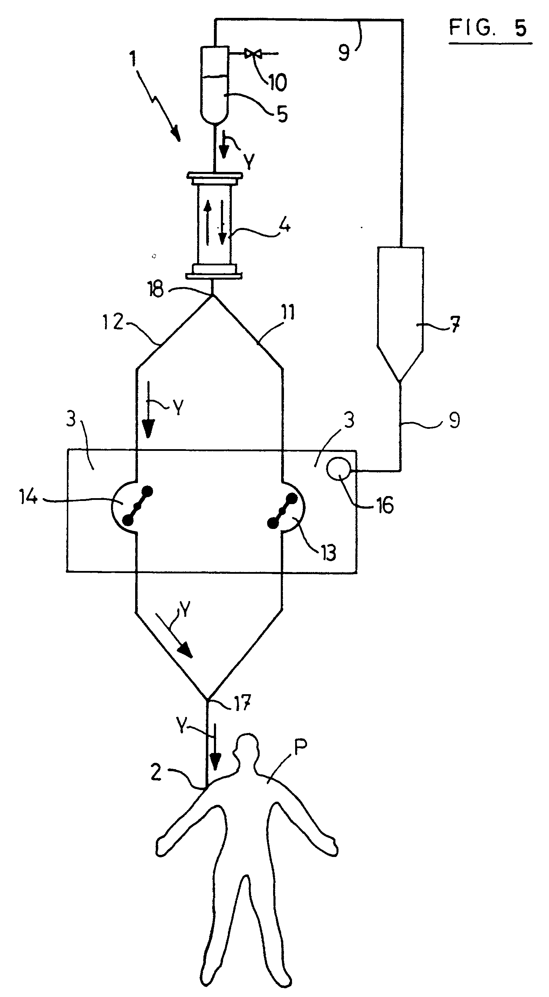

– les figures 4 et 5 illustrent une forme de réalisation alternative de l'appareillage de dialyse selon l'invention.

Dans ces figures, les mêmes signes de référence désignent des éléments identiques ou analogues.

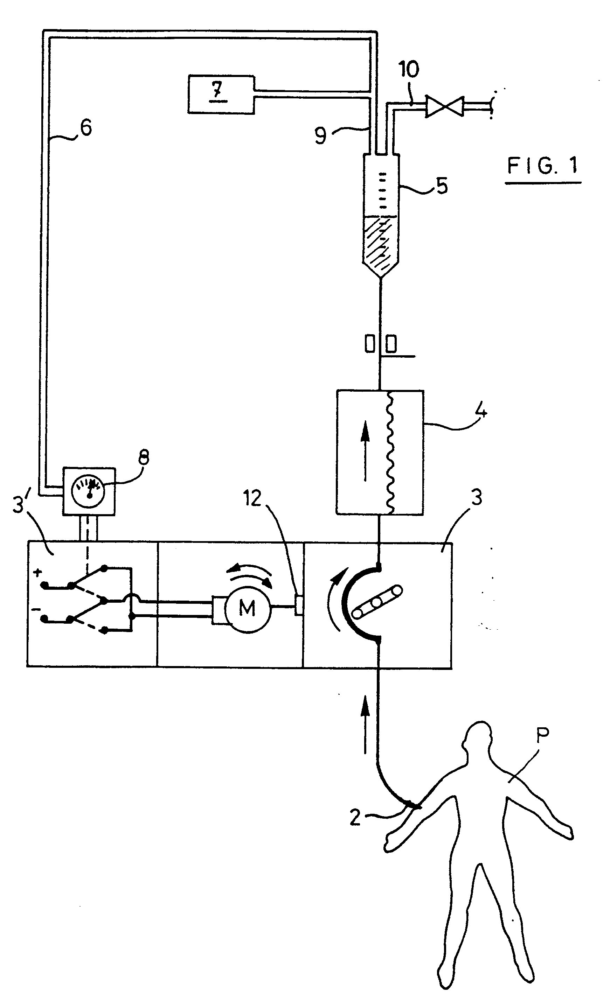

Comme illustré à la figure 1, l'appareillage de dialyse, désigné dans son ensemble par le signe de référence 1, comporte :

* une aiguille 2 du type Deseret Angiocath 14 GA 1/4;

* une ligne sanguine appropriée du type BL 174 montée entre l'aiguille susdite 2 et la pompe citée ci-dessous ;

* une pompe 3 du type BL 760 N ou du type BL 705 transformée en pompe à sens réversible ou une pompe WATSON MARLOW, du type 502 à tête transformée et dispositif de l'inversion du sens de rotation 3' ;

* un rein artificiel 4 de faible volume, par exemple un rein artificiel à fibres creuses ;

* une chambre d'expansion artérielle 5 jouant le réservoir sous pression pour l'accumulation de sang ;

* une ligne de pression du type BL 374 + BL 049 avec une chambre de grande contenance pour l'air et une chambre de compliance incluse ;

* un manomètre 8 pour le contrôle du sens de rotation et de fonctionnement de la pompe, du type NOVO SARA avec valeur de consigne maximale et minimale.

La chambre d'expansion artérielle 5 (AEC) comporte une chambre cylindrique, se terminant en dessous par un fond conique déboutissant dans la conduite reliant celle-ci au rein artificiel 4, et se terminant au-dessus par deux lignes d'air 9 et 10 dont l'une est apicale et l'autre 10 implantée légèrement plus bas ; la ligne supérieure est une ligne de pression et la ligne inférieure une ligne d'infusion 10 servant au rinçage du rein, l'administration de médicaments, réglage de niveau de sang au moyen d'injection d'air.

Dans une forme de réalisation préférée, la chambre présente un diamètre de 35 mm et une hauteur de 20 cm. Elle est constituée généralement de PVC rigide ou d'un autre matériau biocompatible indéformable rigide transparent.

Le sang s'écoule à travers environ 10.000 capillaires. Ce liquide de régénération s'enroule en sens opposé au travers de la gaine en matière plastique et rince les fins tubes, dont les parois servent de membranes semi-perméables.

Cette forme possède l'avantage d'un volume de sang minimal, d'une perte de pression faible et d'une efficacité maximale.

Le sang s'écoule hors du rein artificiel 4 par la partie inférieure dans la chambre d'expansion artérielle 5.

La phase artérielle se poursuit et le sang est accumulé dans la chambre artérielle d'expansion 5 jusqu'à ce que le volume soit 4 à 5 fois supérieur au contenu du rein artificiel 4 en de la ligne sanguine artérielle 3, à savoir environ 150 ml.

La capacité d'extension du volume en variation sous pression, qui est nécessaire pour accumuler le sang, s'obtient par le fait de comprimer l'air dans la partie supérieure de la chambre d'expansion, dans un réservoir d'air d'environ 650 à 2000 ml désigné sous le nom de chambre de compliance 7 assimilée à la ligne de pression 6.

Lorsque la commande de la pompe bidirectionnelle s'effectue par un mécanisme de pression, la pression est enregistrée dans la chambre d'expansion au-dessus du rein artificiel ; à des valeurs de consigne minimale et maximale de la pression dans ce mécanisme, la pompe change de direction et achève ou commence le cycle.

Une valeur de consigne de pression maximale n'est introduite qu'au moment ou la chambre d'expansion artérielle 5 est complètement remplie à quelques centimètres près et la pompe sanguine est inversée ; la chambre 5 est alors vidée par le bas ; lorsqu'elle est presque complètement vide, on introduit la valeur de consigne de pression minimale et la pompe 3 s'inverse à nouveau de manière à recommencer le cycle.

Cette chambre artérielle 5 se remplit et se vide donc par l'orifice unique ménagé dans le fond conique.

Dès au'une pression maximale préalablement choisie est atteinte, le sang est chassé de la chambre d'expansion 5 vers le rein artificiel 4, s'écoule une seconde fois au travers du rein artificiel 4 et est retourné en sens inverse sous l'action de la même pompe, dans la même ligne sanguine dans le corps du patient.

La phase veineuse de refoulement est poursuivie, jusqu'à ce que la pression dans la chambre d'expansion susdite 5 descende sous une pression minimale.

La chambre de compliance qui est montée dans la ligne de pression 7, est nécessaire pour permettre une accumulation suffisante de sang dans la chambre d'expansion 5, sans que l'amenée et l'évacuation de sang ne provoque de grandes différences de pression sur le manomètre 8 ; ceci engendrerait de grands problèmes dans les fluctuations de pression, néfastes pour le réglage de l'ultrafiltration au travers du rein artificiel, le déplacement d'eau au travers d'un rein artificiel 4 dépend en effet de la pression de la membrane d'échange, qui elle-même est fonction de la pression dans le compartiment sanguin du rein artificiel. Des fluctuations dans ce dernier compartiment provoquent des phénomènes d'ultrafiltration incontrôlables. Ces phénomènes sont évités par une chambre de compliance suffisamment dimensionnée que pour permettre que les fluctuations de pression entre le remplissage minimal et maximal de la chambre d'expansion 5 soient minimales ; de ce fait il est possible d'obtenir :

    – une ultrafiltration homogène, et

    – une grande variabilité dans la consignation de pressions préprogrammées, puisque l'échelle de pression n'est pas occupée par une pression de consigne déterminée, mais par une fraction seulement.

La chambre de compliance 7 peut être constituée de n'importe quelle chambre rigide d'environ 650 ml à 2000 ml de contenance, de n'importe quelle forme. Elle peut être insérée dans la ligne de pression ou peut être montée avant le manomètre dans la pompe. Elle doit se trouver nécessairement entre le niveau sanguin de la chambre d'expansion 5 et le manomètre 8 ; un filtre bactérien est monté dans la ligne de pression 9 entre la chambre d'expansion artérielle 5 et la chambre de compliance 7.

Une méthode alternative pour commander la pompe est la méthode volumétrique : en prévoyant par exemple une détection par ultrason à hauteur de la chambre d'expansion, on peut permettre à la pompe de changer de sens à une valeur minimale et maximale du degré de remplissage. Ceci peut être prévu à titre de sécurité (c.à.d. on laisse commander la pompe par la pression mais en même temps on effectue une mesure du volume (minimum et maximum) à hauteur de la chambre d'expansion, ceci afin de pouvoir corriger d'éventuelles mesures de pression erronées. Un inconvénient de cette méthode de régulation par volume, consiste à ne pas savoir sous quelle pression on effectue la dialyse, et c'est justement cette pression qui est importante pour l'ultrafiltration. Dans une pompe commandée par pression, cette ultrafiltration est directement proportionnelle à la valeur de consigne de pression.

Ce défaut peut éventuellement être corrigé dans une pompe du type à commande volumérique, en laissant se réaliser l'ultrafiltration par un fonctionnement sous différence constante de pression dans le compartiment sanguin, à condition de régler l'ultrafiltration par une pompe à ultrafiltration disposée dans le compartiment du dialysat. Dans les appareil d'hémodialyse les plus modernes, l'unité de contrôle de l'ultrafiltration est déjà incorporée.

Une troisième possibilité pour réguler une pompe consiste à travailler avec une commande temporelle. En prévoyant un premier intervalle de temps déterminé pour la phase artérielle du cycle et un second intervalle de temps déterminé pour la phase veineuse du cycle. Ce système a le désavantage qu'il engendre des battements de volume irréguliers parce que le sang n'est pas toujours présent avec disponibilité suffisante, par exemple en raison d'une obstruction légère de l'alimentation en sang : ce type de commande pourrait provoquer des problèmes de sous- ou surremplissage de la chambre d'expansion, qui pourraient être corrigés par un contrôle simultané du volume. En principe il doit être possible d'asservir la pompe à une commande dépendante du temps.

L'avantage le plus important de l'hémodialyse bidirectionnelle à une seule aiguille réside dans le fait que le sang s'écoule deux fois au travers du rein artificiel 4. Ce sang est ainsi mieux purifié et filtré que dans les systèmes connus unidirectionnels. L'efficacité du rein artificiel est environ doublée par rapport aux autres méthodes de dialyse, du moins pour certaines molécules difficilement dialysable.

Lors du premier passage au travers du rein artificiel, seuls les substances de poids moléculaire faible sont éliminées en majeure partie, un second passage permet d'obtenir une élimination des substances de poids moléculaire moyen meilleure que les systèmes conventionnels unidirectionnels.

Un deuxième avantage est une simplification de construction et de commande.

Un désavantage important de cette hémodialyse à une seule aiguille est la recirculation dans le système circulatoire du patient de sang non épuré.

La recirculation décroît lorsqu'on diminue la longueur et le contenu des lignes sanguines et du rein artificiel. Cette méthode exige des reins artificiels, des segments de pompe et des lignes sanguines compacts.

Une chambre d'expansion artérielle empêche la formation de mousse et permet un débit continu en droit du rein artificiel. On suppose que dans les procédés de diffusion ou d'hémodialyse, le débit variable améliore le travail du rein artificiel, en modifiant les couches limites à la surface d'échange de la membrane.

Dans le montage illustré à la figure 1, l'appareil d'hémodialyse comprend les dispositifs de sécurité suivants :

    1. deux détecteurs d'air 12 : le premier monté entre la pompe péristaltique 3 et l'aiguille 2 et le second monté entre la chambre d'expansion artérielle 5 et la pompe péristaltique 3 ;

    2. deux dispositifs de surveillance du nombre de tours de rotation effectués par la pompe 3 sont incorporés dans le segment de pompe en vue de la sécurité : un pour chaque sens de rotation de la pompe, et

    3. un détecteur de surpression et de souspression, monté entre l'aiguille 2 et la pompe 3.

Comme dispositif de sécurité on peut prévoir également des appareils de mesure de niveau capacitifs ainsi que des détecteurs soniques, ultrasoniques ou à cellule photoélectrique.

La pompe 3 peut être constituée :

    – d'une pompe péristaltique à rouleaux ;

    – d'une pompe aspirante faisant intervenir des clapets unidirectionnels grâce auxquels un flux sanguin continu peut être obtenu par le déplacement alternatif d'un piston ;
— d'une pompe à membrane ou d'un souflet élastique

Il est évident que la chambre d'expansion doit être rigide dans le cas d'une pompe asservie à la pression ou d'un mécanisme de refoulement du sang puisque la pression exacte doit pouvoir être mesurée, sans intervention d'autres facteurs, tels que l'élasticité du récipient, dans lequel le sang est accumulé. Dans le cas d'une pompe asservie ou un volume ou à un intervalle de temps ou dans le cas d'un mécanisme de refoulement, on peut utiliser une structure non rigide telle qu'un ballon.

Le montage décrit ci-dessus peut être amélioré en ramifiant la ligne sanguine et en y adjoignant une seconde pompe.

On parvient ainsi à remédier dans certaines conditions à un débit cathéterien ou fistuleux moins satisfaisant.

L'appareillage de dialyse, désigné dans son ensemble par le signe de référence 1 comprend une double ligne sanguine extracorporelle 11, 12 comportant une pompe à double tête 13, 14, un rein artificiel 4 et une chambre d'expansion 5 sous pression.

Le prélèvement du sang à partir du système circulatoire du patient P, se réalise à l'aide d'une aiguille 2 du type Deseret Angiocath 14 GA 1/4.

Un premier embranchement 17 en forme d'Y est prévu à l'extrémité libre de l'aiguille 2 pour raccorder l'aiguille 2 à l'aide d'une double ligne sanguine 11, 12 du type DL 174 à la pompe à double tête 13, 14 par exemple du type BELLCO 760 B.

La pompe à double tête 13, 14 est raccordée au rein artificiel à l'aide d'une seconde pièce 18 en forme d'Y, qui est montée juste à l'entrée du rein artificiel.

Le rein artificiel présente de préférence une contenance plus petite.

La chambre d'expansion artérielle est montée derrière le rein artificiel 4.

La ligne de pression est du type BL 374 ou BL 049. Elle relie la chambre d'expansion artérielle 5 à la chambre de compliance 7 et à un manomètre 16 qui asservit la pompe à double tête.

Le manomètre 16 est par exemple du type NUOVE SARA avec valeurs de consigne maximale et minimale.

La chambre d'expansion artérielle 5 (AEC) est du même type que celle décrite ci-dessus. Elle est formée d'un réservoir cylindrique d'environ 200 ml, aboutissant par le bas coniquement dans une ligne sanguine conduisant au rein artificiel.

La ligne de pression se trouve au-dessus, tandis que la ligne d'infusion est située un peu plus bas et sert à diverses applications.

Pendant la première phase, le sang est prélevé à l'aide de la première pompe 13 et de l'aiguille 2, hors du système circulatoire du patient P et conduit par une multitude de capillaires du rein artificiel 4 à fibres creuses dans le sens de la flèche X vers la chambre d'expansion 5.

La pression dans la chambre d'expansion 5 est réglée au choix. Cette pression détermine la pression de fonctionnement dans le rein artificiel 4 et par conséquent l'ultrafiltration.

La première phase se poursuit jusqu'à ce qu'une pression de consigne préalablement choisie soit atteinte dans la chambre d'expansion 5. L'augmentation de pression est la conséquence de la compression de l'air en raison de remplissage de la chambre d'expansion par du sang.

Ce manomètre 16 commande la pompe à double tête 13, 14 et veille à l'alternance du fonctionnement de celle-ci comme dans le système déjà connu de la pompe à double tête de Van Waeleghem et Ringoir. Dès que la chambre d'expansion 5 est remplie à un niveau convenable et la pression maximale atteinte, la première pompe 3 est désarticulée et bloque ainsi la ligne sanguine artérielle.

La deuxième pompe 14 est immédiatement enclenchée.Elle est dirigée dans un sens opposé à celui de la première pompe 13 et pompe pendant la deuxième phase le sang hors de la chambre d'expansion 5, au travers du rein artificiel 4 et le long de deux embranchements 17, 18 et l'aiguille 2 dans le système circulatoire du patient P. La deuxième phase est poursuivie jusqu'à ce que la pression descende dans la chambre d'expansion 5 en-dessous d'une pression de consigne préalablement choisie minimale.

Pendant le cycle complet, le sang s'écoule deux fois de suite, en sens opposé au travers du rein artificiel 4.

Dans cette seconde forme de réalisation, l'avantage le plus important de l'hémodialyse bidirectionnelle à une seule aiguille est maintenu. Il consiste à faire passer deux fois le sang dans le rein artificiel.

Ceci permet d'obtenir une meilleure épuration du sang et une efficacité accrue de l'appareillage.

Un avantage supplémentaire de la seconde forme de réalisation par rapport à la première forme de réalisation réside dans le fait que la recirculation lors de chaque inversion du flux dans la ligne sanguine 11, 12 et les segments de pompe compris entre l'aiguille 2 et le rein artificiel 4 est éliminée grâce à l'insertion d'une seconde pompe et grâce à la scission de la ligne sanguine en deux embranchements 11, 12.

La simplicité de l'appareillage 1 est perdue en partie mais de ce fait la recirculation qui constitue le principal inconvénient du système susdit de l'hémodialyse bidirectionnelle à une seule aiguille, est complètement éliminée.

La recirculation peut être négligée, quelque soit la longueur et le contenu des lignes sanguines 11, 12 en des segments de pompe.

Les avantages apportés par la chambre d'expansion artérielle 5 dans le système d'hémodialyse à une seule aiguille du type pression-pression du type Dr. RINGOIR sont maintenus tant dans la première que dans le seconde forme de réalisation.

La chambre d'expansion artérielle 5 forme un réservoir destiné à permettre l'accumulation de sang entre chaque inversion du flux. Elle sert à enregistrer la pression qui commande le fonctionnement alternatif de la pompe double 13, 14.

Cette chambre est immédiatement montée sur le rein artificiel 4 au lieu d'être intégrée dans la ligne sanguine. Le raccordement de la chambre 5 et du rein artificiel s'effectue aussi parfois à l'aide d'un segment aussi court que possible.

Le fonctionnement alternatif de l'appareil de dialyse s'obtient grâce à un programme de commande simple qui permet aux proches parents d'effectuer la dialyse.

Ce système convient particulièrement aux enfants, mais aussi aux adultes qui connaissent des difficultés de débit sanguin insuffisant au travers de l'aiguille ou de la fistule.

Comme dispositif de refoulement, on peut utiliser une pompe à double tête ou un système à clapets selon KOPP telle que décrite dans le documents US 4643714.

Pour augmenter l'efficacité de la dialyse, on peut modifier les conditions opératoires du côté du dialysat, de la manière suivante :

1. accélérer le flux de dialysat dans le rein artificiel, en réduisant les dimensions du compartiment du rein artificiel destiné au dialysat ou en augmentant le flux de dialyse jusqu'à 750 à 1000 ml/min ;

2. prévoir pour chaque inversion du flux du sang dans le rein artificiel, une invention simultanée du flux de dialysat à l'aide d'un dispositif d'invention.

Un dispositif avantageux d'inversion comporte des conduits d'amenée et d'évacuation de dialysat disposés parallèlement entre eux et scindés de manière qu'une seule vanne puisse obturer une paire de conduits droits et une paire de conduits gauches. En asservissant ces vannes à celles qui règlent l'écoulement du sang, on veille à ce que les flux de sang et de dialysat soient toujours parallèles ou opposés. On peut même choisir un type particulier d'écoulement en fonction de rendre sélectif le travail d'épuration du rein des substances de faible poids moléculaire ou de poids moléculaire plus élevé.

Il est évident que l'invention n'est pas limitée à la forme de réalisation décrite ci-dessus et que de nombreuses modifications peuvent être apportées à celle-ci sans sortir du cadre de l'invention, tel que défini par les revendications.

Ainsi, l'appareillage d'hémodialise selon l'invention peuvent aisément s'appliquer à l'hémofiltration.

**Revendications**

1. Appareillage pour l'hémodialyse à une seule aiguille (2), dans lequel un volume prédéterminé de sang provenant d'une fistule, d'une veine ou d'une artère est alternativement retiré du corps du patient (P) au moyen d'une pompe (3, 13, 14) pour être épuré dans un rein artificiel (4) et ensuite retourné au patient au même endroit, comportant :
   – une aiguille ou un cathéter (2),
   – un tronçon de tuyau entre l'aiguille (2) et la pompe (3, 13, 14),
   – le rein artificiel (4)
   – une chambre d'expansion (5) constituée d'un récipient fermé pourvu d'une conduite d'amenée et d'évacuation du sang et muni à la partie supérieure d'une conduite apicale d'air (9) et munie quelques millimètres plus bas d'une ligne d'infusion (10) permettant de rincer le rein (4), d'administrer un médicament et d'adapter la hauteur du niveau de sang à l'aide d'air sous pression, et
   – un dispositif de mesure et de contrôle (16) de la pompe (3, 13, 14), caractérisé en ce que la pompe (3, 13, 14) est réversible ;
   – l'aiguille (2), la pompe (3, 13, 14) et la chambre d'expansion (5) sont situées sur une même ligne sanguine ;
   – la chambre d'expansion (5) est plus éloignée du point de ponction que la pompe (3, 13, 14) ;
   – – la ligne d'infusion (10) aboutit dans la chambre d'expansion (5) quelques millimètres plus bas que la conduite apicale d'air (9).

2. Appareillage selon la revendication 1, caractérisé en ce qu'une chambre d'expansion (5) est pourvue d'un seul orifice ou conduit destiné à amener ou retourner du sang.

3. Appareillage selon la revendication 1 caractérisé en ce qui la ligne sanguine (6) lorsqu'on s'éloigne du point de ponction, de la pompe (3) et du rein artificiel (4) aboutit sur la chambre (5) d'expansion qui est reliée à une chambre de compliance (7) par une ligne de pression (9).

4. Appareillage selon l'une quelconque des revendications précédentes, caractérisé en ce que le rein artificiel (4) est relié d'un côté à l'aide d'une pompe (3) et une seule aiguille (2) ou cathéter au système circulatoire du patient (P) et de l'autre côté relié à une chambre d'expansion (5) comprenant un récipient muni d'une conduite d'amenée et d'une conduite d'évacuation du sang, et à la partie supérieure, d'une conduite apicale (9) d'air destinée à jouer le rôle d'une ligne de pression, et quelques millimètres plus bas, d'une ligne d'infusion (10) pour le rinçage du rein, l'administration de médicaments et le réglage du niveau du sang à l'aide de la quantité d'air injectée.

5. Appareillage selon l'une quelconque des revendications précédentes, caractérisé en ce que la

8

chambre d'expansion (5) et la chambre de compliance (7) comportent un dispositif de mesure de pression (16).

## Ansprüche

1. Vorrichtung für die Blutdialyse mit einer einzigen Nadel (2), durch die ein vorgegebenes Volumen an Blut alternativ aus einer Fistel, einer Vene oder einer Arterie des Patientenkörpers (P) vermittels einer Pumpe (3, 13, 14) abgezogen wird zur Reinigung in einer künstlichen Niere (4) und anschließend an der gleichen Stelle in den Patienten zurückgeführt wird, mit :
   – einer Nadel oder einem Katheter (2),
   – einem Schlauchstück zwischen der Nadel (2) und der Pumpe (3, 13, 14),
   – einer künstlichen Niere (4),
   – einer Expansionskammer (5), bestehend aus einem geschlossenen Behälter, der mit einer Zuleitung für das Zuführen und beziehen von Blut versehen und am oberen Ende mit einer oberseitigen Luftleitung (9) und einige Millimeter tiefer mit einer Infusionsleitung (10) ausgestattet ist, um das Spülen der Niere (4), das Verabreichen eines Medikaments und das Einstellen der Höhe des Blutspiegels mit Hilfe von unter Druck stehender Luft zu ermöglichen, und
   – einer Meß- und Steuereinrichtung (16) für die Pumpe (3, 13, 14), **dadurch gekennzeichnet, daß**
   – die Pumpe (3, 13, 14) umkehrbar ist,
   – die Nadel (2), die Pumpe (3, 13, 14) und die Expansionskammer (5) an einer gemeinsamen Blutleitung angeordnet sind,
   – die Expansionskammer (5) weiter von der Punktionsstelle entfernt liegt als die Pumpe (3, 13, 14),
   – die Infusionsleitung (10) einige Millimeter unterhalb der oberseitigen Luftleitung (9) in die Expansionskammer (5) einmündet.

2. vorrichtung nach Anspruch 1, **dadurch gekennzeichnet,** daß die Expansionskammer (5) mit einer einzigen Öffnung oder Leitung versehen ist, die zum Zu- oder Abführen von Blut bestimmt ist.

3. vorrichtung nach Anspruch 1, **dadurch gekennzeichnet,** daß die Blutleitung (6) von der Punktionsstelle ausgehend, über die Pumpe (3) und die künstliche Niere (4) verlaufend in die Expansionskammer (5) einmündet, die über eine Druckleitung (9) mit einer Ausgleichskammer (7) verbunden ist.

4. vorrichtung nach einem der vorhergehenden Ansprüche, **dadurch gekennzeichnet,** daß die künstliche Niere (4) auf einer Seite mittels einer Pumpe (3) und einer einzigen Nadel (2) oder einem Katheter mit dem Kreislauf des Patienten (P) und auf der anderen Seite mit einer Expansionskammer (5) verbunden ist, die einen Behälter mit einer Leitung zum Zuführen und einer Leitung zum Abziehen von Blut, und am oberen Ende eine oberseitige Luftleitung (9) aufweist, die als Druckleitung dient, und wenige Millimeter tiefer eine Infusionsleitung (10) zum Spülen der Niere, zur verabreichung von Medikamenten und zum Steuern des Blutspiegels mit Hilfe der eingeleiteten Luftmenge aufweist.

5. vorrichtung nach einem der vorhergehenden Ansprüche, **dadurch gekennzeichnet,** daß die Expansionskammer (5) und die Ausgleichskammer (7) eine Druckmeßeinrichtung (16) enthalten.

## Claims

1. Apparatus for hemodialysis using a single needle (2), in which a predetermined volume of blood from a fistula, a vein or an artery is alternately drawn from the body of the patient (P) by means of a pump (3, 13, 14) to be filtered in an artificial kidney (4) and then returned to the patient (P) at the same puncture location comprising :
   – a needle or a catheter (2),
   – a section of tube between the needle (2) and a pump (3, 13, 14),
   – the artificial kidney (4),
   – an expansion chamber (5) composed of a closed receptacle provided with a blood delivery and removal tube and provided in its upper part with an apical air tube (9) and provided, a few millimeters lower, with an infusion line (10) enabling the kidney (4) to be rinsed, medication to be administered and the height of the blood level to be adjusted by means of air under pressure, and
   – a device (16) for measuring and controlling the pump (3, 13, 14), characterized in that
   – the pump (3, 13, 14) is reversible,
   – the needle (2), the pump (3, 13, 14) and the expansion chamber (5) are situated on a same blood line,
   – the expansion chamber (5) is farther from the puncture point than the pump (3, 13, 14),
   – the infusion line (10) ends in the expansion chamber (5) a few millimeters lower than the apical air tube (9).

2. The apparatus in accordance with claim 1, characterized in that the expansion chamber (5) is provided with a single orifice or channel intended to deliver or return the blood.

3. The apparatus in accordance with claim 1, characterized in that the blood line (6), going from the puncture point, the pump (3) and the artificial kidney (4), ends in the expansion chamber (5) which is connected to a compliance chamber (7) by a pressure line (9).

4. The apparatus in accordance with any one of the preceding claims, characterized in that the artificial kidney (4) is connected on one side by means of a pump (3) and a single needle (2) or catheter to the circulatory system of the patient (P) and is connected on the other side to an expansion chamber (5) containing a receptacle provided with a blood delivery tube and a blood removal tube, and in its upper part an apical air tube (9) intended to act as a pressure line and, a few millimeters lower, an infusion line (10) for rinsing the kidney, administering medication and adjusting the level of the blood by means of the amount of air injected.

5. The apparatus in accordance with any one of the preceding claims, wherein the expansion chamber (5) and the compliance chamber (7) contain a device for measuring pressure (16).

F I G. 1

F I G. 2

FIG. 3

FIG. 4

FIG. 5